(19) Europäisches Patentamt
European Patent Office
Office européen des brevets

(11) EP 4 527 362 A1

(12) **EUROPEAN PATENT APPLICATION**

(43) Date of publication:
26.03.2025 Bulletin 2025/13

(51) International Patent Classification (IPC):
A61F 9/007 (2006.01)     A61F 9/008 (2006.01)

(21) Application number: 23198979.9

(52) Cooperative Patent Classification (CPC):
A61F 9/008; A61F 9/0079; A61F 2009/00863

(22) Date of filing: 22.09.2023

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: OD-OS MacuTherm GmbH
14513 Teltow (DE)

(72) Inventors:
• Rolfs, Janine
  15711 Königs Wusterhausen (DE)
• Hoff, Annemarie
  14469 Potsdam (DE)
• Rose, Arnd
  14532 Stahnsdorf (DE)

(74) Representative: Pfenning, Meinig & Partner mbB
Patent- und Rechtsanwälte
Joachimsthaler Straße 10-12
10719 Berlin (DE)

(54) **DEVICE AND METHOD FOR RETINAL TREATMENT ACCORDING TO PREDEFINED AREA SECTIONS**

(57)     The invention relates to a method and to a device for tissue treatment of the retina (16) of a patient's eye (4) by means of a laser (2) controllable with respect to one or more irradiation parameters, wherein the laser beam (11) can be directed to different areas of the retina, wherein the device is adapted to assign irradiation parameters to selected treatment area regions (20a, 20b, 20c) of the retina and to irradiate these treatment area regions using the irradiation parameters, wherein each treatment area region is associated with one of a plurality of mutually adjacent, predefined area sections (n1, s1, t1, i1, n2, s2, t2, i2) which together may cover an area of the retina surrounding the fovea (21) the extent of which is such that it does not cover the exit site of the optic nerve (22) and in particular the arcades (23, 24).

Fig. 1

EP 4 527 362 A1

**Description**

The invention is in the field of medical technology, in particular ophthalmology, and can be used with particular advantages in laser eye treatments.

Eye treatments by means of lasers have become known for a variety of diseases and for the pursuit of various goals. In addition to the removal or ablation of the corneal lens of the eye, treatments of the retina using a laser are particularly well known. In such treatments, the effect is largely based on the controlled input of energy in the form of light energy into the retina or tissue volumes close to the retina, in order to achieve for example the coagulation of cells or other tissue changes by increasing the temperature of the tissue for a limited period of time. In some applications, this can even go as far as the targeted thermal destruction of cells.

Laser treatments of the eye of a patient must be planned diligently because of the existing risks and the complex tasks in order to prevent or minimize damage to treated tissue regions as well as regions that are not to be treated. For this reason, such treatments are carried out as far as possible by physicians or under the close supervision of physicians.

In many cases, prior to retinal treatment, an image of the retina of the eye potentially to be treated is recorded and a decision is made as to the necessity and/or type of treatment based on conditions that are recognizable by means of the image.

From the European patent application EP 1875 857 A1 an ophthalmoscope with an imaging optics and an illumination device as well as an optical sensor is known, with which images of the retina of an eye can be recorded. Such an ophthalmoscope can be used, for example, for planning a laser treatment by a physician.

Against the background of the state of the art, the present invention is based on the task of simplifying the planning and performance of retinal treatments.

The task is solved by the invention with the features of the independent patent claims. The dependent patent claims present advantageous implementations of the invention.

Accordingly, the invention relates to a device for tissue treatment of the retina of a patient's eye by means of a light source, in particular a laser controllable with respect to one or more irradiation parameters, wherein a light beam emitted by the light source can be directed to different areas of the retina, wherein the device is arranged to assign irradiation parameters to selected treatment area regions of the retina and to irradiate these treatment area regions using the irradiation parameters, wherein each treatment area region is associated with one of a plurality of mutually adjacent, predefined area sections.

He predefined area sections together may cover a composed area of the retina surrounding the fovea and in some cases excluding the fovea, the extent of the composed area being such that it does not cover the exit site of the optical nerve and in particular the arcades.

In this context, the arcades are understood to be the main supply arteries of the retina originating directly from the site of the exit point of the retinal artery in the retina, which surround the fovea and the retinal area adjacent to it over a wide area.

The retina of a patient's eye can be treated over a wide area by serial illumination of laser spots, in which light energy is introduced into the target tissue by localized illumination at each spot, thus heating the tissue locally in a targeted manner. Tissue heating can lead to immediate tissue changes by thermally destroying the tissue or by heating it above a coagulation temperature. However, it has been found that a moderate temperature increase can also initiate or accelerate non-destructive processes that can bring about certain physiological processes, including healing processes.

Particularly when non-destructive processes are targeted, treatment area regions can be selected and treated relatively generously. In this case, uniform irradiation parameters can be applied within the treatment area regions, or profiles of irradiation parameters can be applied, even if in some cases, separate irradiation parameters can be defined for each individual laser spot.

In many cases, a treatment plan for a retina results in locally distributed treatment area regions that are to be irradiated successively by a treatment laser.

In order to enable optimized planning and control of the treatment laser or, more generally, a light source, for retinal treatment in the sense of optimized logistics, area sections are predefined to which the treatment area regions are assigned. This makes it easier, more reliable and more reproducible to identify and locate the treatment area regions on the retina in which the retina is to be treated and to address and cover them with a light source, in particular a treatment laser. The treatment laser can also be controlled more easily with reference to the predefined area sections. The treatment area regions can be freely positioned on the retina, but they can be easily and quickly located by associating them with the predefined area sections. #the light source, apart from a laser, may also, for example, be realized as a light emitting diode (LED)

A further advantage of the use of predefined area sections is that they allow a standardization for location information on the retina, so that, for example, treatment area regions can be defined by means of a first system and the location of the treatment area regions can be found and addressed by means of another system and/or at a later point of time. An already known exemplary system of such predefined area sections is given by the so-called ETDRS grid, which is already used in the area of the macula diagnosis to categorize diagnostic information, the results of functional tests and, for example, also the results of layer thickness measurements by optical coherence tomography.

Advantageously, the device can be set up in such a way that it is arranged for the light source, in particular a laser, to successively irradiate the predefined area sections or treatment area regions on the predefined area sections in a predetermined order or according to a predetermined rule.

The predetermined sequence or a rule according to which a sequence can be determined can thereby be set up, for example, in such a way that the logistics of the irradiation or the load or utilization of the irradiation device can be optimized. A potential rule could for example be that the mutually adjacent area sections are treated in a fixed sequence and, if one and not more than one single area section in the predetermined sequence is not selected for treatment, it will not be skipped and will be treated anyway, whereas, if at least two mutually adjacent area sections in the sequence are not selected for treatment, they are skipped.

The word "laser" is used here and in the following to refer, for example, to an ophthalmological treatment laser. The light source for treatment may, however, also be a different one, like a light emitting diode.

By assigning the treatment area regions to the predefined area sections, the treatment area regions can be easily addressed with the light source, in particular a treatment laser. A sequence of irradiations can also be defined in advance according to the assignment to the predefined area sections, so that the changes in setting, for example beam angle changes, of the laser from area section to area section and thus also from one treatment area region to the next can be minimized. This allows irradiation inaccuracies to be minimized, as well as the time required for the overall treatment.

A further embodiment of the invention can provide that the light source control, in particular the laser control, is set up to irradiate a group of area sections, in particular all area sections individually one after the other, in each case either not covering an area section at all or covering 100% of the area section.

Thus, for example, complete area sections can be defined as treatment area regions or as areas to be excluded and subsequently treated or not treated with the laser or light source. The irradiation parameters can then also be chosen uniformly for entire area sections, for example.

The entire retinal areas, each belonging to an area section, can be pre-programmed in the laser control unit, so that in the case of treatment, filling an area section with laser spots in the course of pre-programming the laser settings is no longer necessary, but has already been done or can at least be easily retrieved from a data storage device.

A further advantageous embodiment of the invention can, for example, provide that at least one group of area sections are arranged next to one another in the circumferential direction around the fovea.

This setup is particularly useful because it provides a clear arrangement of the area sections, and the area sections immediately surrounding the fovea are also the most important areas for a patient's vision, since the areas of sharpest optical perception are located in the immediate vicinity of the fovea.

Another useful arrangement may provide that the area sections are distributed around the fovea, in particular in the form of segments of at least one circular ring concentrically surrounding the fovea or of an elliptical ring surrounding the fovea.

It may also be provided that the area sections together cover an area of the retina surrounding the fovea the extent of which is in particular such that it does not cover the exit site of the optic nerve and in particular the arcades.

For example, it can also be advantageously provided that the area sections are arranged on one or more rings, in particular circular rings, each of them concentrically surrounding the fovea, the area sections being arranged rotationally symmetrically with an n-count rotational symmetry, where n is in particular between 1 and 9 and is further in particular 2, 4 or 6.

It can be further advantageously provided that the area sections form ring segments of two or three concentric circular rings.

Thus, in such a solution, the segments are categorized in different distance ranges from the fovea, the relevance of which for treatment can decrease, for example, with increasing distance from the fovea.

It can be further provided, for example, that the irradiation intensity of the laser is controlled in such a way that the power density in the treatment area regions irradiated by the laser is between 100 W/cm$^2$ and 350 W/cm$^2$, in particular between 120 W/cm$^2$ and 300 W/cm$^2$, wherein the irradiation time for each laser spot in a treatment area region remains less than 500 msec (milliseconds), in particular less than 200 msec.

It may further be provided that the period of irradiation of a single laser spot with said power is at least 10 msec, further in particular at least 50 msec, further in particular at least 100 or 150 msec.

For example, time periods between 50 and 200 msec or between 50 and 500 msec or between 100 and 200 msec or between 100 and 500 msec may thus result.

With the limited irradiation intensity described, it is ensured that no immediate tissue damages or changes are caused by the laser irradiation, but it is nevertheless achieved that certain physiological processes, such as the release of various enzymes, hormones and messenger substances, is caused or accelerated, which have a positive effect on the health of the target tissue. This may result in slowing down the progression of various retinal diseases or aging processes. Treatment at the indicated irradiation intensities is painless and cannot cause any harm. Therefore, no harm is to be feared even if treatment area regions are exceeded and it can be decided, for example, to irradiate certain area sections completely and covering the entire area without major risks. This can be useful, for example, if a decision is made to treat the entire area for each area section in which at least one treatment area region to be treated is located. The boundaries of the area sections can be stored in the device, so that the programming of the laser treatment is considerably simplified.

Another possible embodiment of the invention may provide an eye-tracking device that compensates for movements of the eye during irradiation of the treatment area regions and/or by a temperature measurement device comprising a thermo- optic spectrometer which sends laser pulses to the retina and registers pressure waves being emitted by the retina as a response signal.

Once the areas of the retina to be treated are determined and the laser is programmed, any eye movement may cause the retina to move laterally under the laser beam, thereby exposing to the laser beam areas of the retina that are not worthy of treatment or areas that should not be irradiated with the laser.

For this reason, so-called eye tracking is performed, i.e., the movements of the treated eye are detected and the steering of the beam of the treatment laser is adjusted in such a way that the target areas/treatment area regions of the retina are reliably hit despite the eye movement. The eye movement can be detected, for example, either by means of a camera which is directed to the eye or by detecting reflected scanning beams.

The temperature measurement device is based on the "Grüneisen" formula and measures the absorption of laser radiation by the response signal in the form of pressure waves, which are created by the sudden temperature rise in the retina. The temperature measurement device can be used to establish a closed loop with the laser control and reliably limit the temperature at a current laser spot to the target temperature by controlling the laser power.

In a further embodiment, for example, a retinal detection device can be provided which records an image of the predefined area sections of the retina by means of an imaging process and assigns irradiation parameters associated with the individual area sections or treatment area regions arranged thereon.

Such a retina detection device can be used, for example, to detect the intensity of the pigmentation of the retina and to control the power of the treatment laser as a function thereof.

For this purpose, for example, a normalized illumination of the retina with respect to the illumination intensity and/or the illumination wavelength or the illumination spectrum can be provided during the retinal detection. It may also be provided that areas of the retina which have no or very little pigmentation, such as the areas where large blood vessels run or the head of the optical nerve, are used to normalize the pigmentation measurement.

In addition to a device of the type described above, the invention also relates to a method for tissue treatment of the retina of an eye of a patient by means of a light source, in particular a laser controllable with respect to one or more irradiation parameters, wherein the laser beam can be directed to different areas of the retina, wherein firstly treatment area regions are defined or determined in which laser irradiation is useful and thereafter the treatment rules, in particular the treatment order of treatment area regions are determined according to the assignment of the treatment area regions to predefined area sections of the retina, wherein the predefined area sections are mutually adjoining one another and together covering a composed area surrounding the fovea, the extent of the composed area being dimensioned in such a way that it does not cover the exit location of the optic nerve and in particular the arcades. The composed area surrounding the fovea may in some cases exclude the fovea itself.

In such a method, the assignment of the treatment area regions to predefined area sections allows a logistically simplified and optimized control of the laser during the treatment and thus an acceleration of the treatment and in many cases also a simplified and accelerated treatment planning. In addition, both the treatment planning and the treatment are less prone to errors, especially when all or some area sections are treated either completely or not at all.

The method can be advantageously designed, for example, in that an image of the predefined area sections of the retina is recorded by an imaging method and irradiation parameters are assigned to the individual area sections or to treatment area regions defined on these.

In this case, it is easy to determine whether entire area sections are to be treated at 100% or not at all, or whether irradiation parameters are to be set in a differentiated manner. When determining the irradiation parameters, detected diseases or other anomalies of the retina can be taken into account. Likewise, when determining optimized irradiation parameters, the degree of pigmentation of the retina in the area covered can be taken into account. The irradiation parameters can be understood in particular as the length and intensity of the laser irradiation, but also other laser parameters such as the pulse frequency and the duty cycle and the degree of focusing of the laser spot, i.e. also the size of the laser spot and the power density.

A further embodiment of the method according to the invention can also provide that during the irradiation of the treatment area regions a movement of the eye is compensated by an eye-tracking method.

The eye-tracking method has already been described in detail above.

The device and method may also include a device and method for generating planning data sets for tissue treatment of the retina of a patient's eye using a laser,

- comprising a camera adapted to continuously capture images of the retina,
- with an image processing device which is arranged to generate and provide current structured image data of the retina from images captured by the camera,
- with an inventory data acquisition device which is arranged to acquire and/or provide inventory data of a patient,

and with a processing device which is arranged to use the current structured image data of the retina and the inventory data of the data sets for the tissue treatment, wherein specifications, in particular patterns and rules, for the assignment of planning data to image data and inventory data of patients are stored in the processing device.

In addition, it can be provided that the system has a modification device, which can also be referred to as a correction device, and which enables manual modification of a planning data record. Such manual changes can be made during or especially after the creation of a planning data set by an expert based on his experience or additional knowledge.

The creation of planning data and also the application of the correction device can be based on the predefined area sections. For example, irradiation parameters can be defined uniformly for each predefined area section, or a procedure, for example a sequence, can be defined for the treatment according to the area sections.

The device or a system including the device or a method can in some cases be set up to support the creation of a treatment plan, which is created in the form of a planning file or planning data, before the treatment of the retina of a patient begins. A prerequisite for this is that the system comprises a camera or, more generally, an imaging system adapted to continuously capture images of the retina. In one embodiment, the imaging system may be locally separate from the treatment device and the optical image may also be created independently at a time prior to treatment. A camera for the creation of the optical image can be designed as an optical colour, infrared or black-and-white camera or as a scanning system, or can also have various filter options, and enables the acquisition of current images of the retina before and/or during planning data are generated. This can be important, for example, in order to be able to compare other captured or currently determined data of the retina with the currently captured state and positioning of the retina in the system. In the context of the present invention, structured graphic or image data are to be understood as all preparations of pure pixel data and the data derived from these preparations or directly from the pixel data, in particular so-called vector-oriented image data. In this context, for example, the location and size of blood vessels, the location of the optic nerve head, and the location, size, and type of drusen, namely soft drusen, hard drusen, and reticular pseudo-drusen (RPD), can be provided as structured image data and taken into account in the creation of planning data sets.

For example, if patient inventory data is used, it may be in the form of a diagnosis or indication, or images of the patient's retina taken earlier by the system itself or by another imaging facility. These should then be able to be compared in the system or the device according to the invention with a current image. Inventory data can also be data from a treatment previously performed on the patient or data on a treatment status. In principle, inventory data can be all data of the patient already available in written form or in electronically, in particular digitally, stored form before the start of treatment or before the start of the creation of the planning data record. This data can be transmitted to the device or a system via electronic interfaces or entered manually. The inventory data can, for example, be available in electronic form in a comprehensive system, of which the system for creating planning data records according to the invention represents a subsystem. The inventory data can also be collected or attributed to or stored according to the predefined area sections.

An advantage of a camera that captures actual images of the retina arises when a device or system for creating planning data is combined with a treatment device that has a treatment laser that is adjusted relative to or aligned with the camera in order to perform the treatment using the planning data. In this case, the matching of the area sections to be treated and/or the treatment area regions arranged on them between the recording and the treatment is particularly simple and reliable.

Furthermore, an image processing device can be provided which generates structured image data of the retina from the currently acquired image data of the camera and provides these for the planning. Structured image data means, for example, data that contain or represent certain objects detected in the images by the image processing, such as the fovea, or properties of the image or the retina, or properties of image areas or parts of the retina, such as the predefined area sections, for example of an ETDRS grid. For example, the structured image data can describe brightness or colour distributions, intensity distributions on an infrared image, the position, shape and/or gradients of objects or object boundaries. The representation of such structures in the form of data can be diverse and include, for example, pure image data, but also vectorial representations, vectors, matrices or other types of abstracted representations. For example, the structured data should describe in a clear form, for example also vector-oriented or with metadata referring to geometric shapes, the shape, position and possibly other parameters of blood vessels, nerve nodes and generally of abnormalities on the retina.

The location of such objects that are detected on the retina, such as blood vessels, the optical nerve head or drusen, can be assigned to predefined area sections and should be taken into account, if necessary, when creating a treatment plan, in particular when locating the treatment area regions on the predefined area sections of the retina.

It may be envisaged in a retinal treatment device or planning data generation device or system that certain elements of the device or system are located at the location where the patient is to be treated, while other elements are located elsewhere or are difficult to locate. For example, the image processing device or even the processing device for generating planning data or parts of this device, such as storage devices, may be located at a different place than the treatment laser. For example, data processing facilities may be located in larger computers or data processing facilities than are available at the location of the laser treatment. If the processing facilities need access to a lot of stored data, both the processing facilities and storage facilities may be distributed across multiple computers or located in a larger, central computer facility. For example, it may be useful to store treatment data from many patients in the form of a database centrally or in a distributed computer system, in order to have as much data as possible available for the system to access when interpreting individual data from a single patient, or for training a learning facility of the system.

Individual parts or modules of the system, such as the inventory data acquisition device, a measurement data acquisition device or a change device for manual correction of planning data or also a device for inputting data describing the treatment success, can also be installed as an application on a mobile terminal, for example. The transmission of input data to the mobile device and the output from the mobile device to a stationary part of the system, which also contains the camera and/or the processing device, is then carried out via interfaces in which both the data formats and the data transmission paths, such as hardware connections, data bus or radio interface, are clearly defined in order to obtain an integrated system.

One embodiment of such a system can, for example, provide that a patient is assigned one of several predefined patient categories on the basis of inventory data and/or currently acquired data, and that specifications of the processing device, in particular an applied laser power or patterns and rules, for the assignment of planning data to currently acquired data and inventory data are determined or influenced by the respectively assigned patient category.

The patient category assigned to the respective patient can depend, for example, on stored inventory data, such as age, certain physiological parameters of the patient, such as weight, type and strength of skin pigmentation, hair colour, pigmentation of the iris, the history of the disease and on the type, intensity and success of preventive treatments performed and/or on currently measured or acquired data, such as structured retinal image data or currently acquired physiological data and/or parameters of the patient, which may be measured in or on the eye or may be independent of parameters of the eye, such as age of the patient, eye colour, hair colour, skin colour, skin pigmentation intensity, body build and current health status. The patient category can be used, for example, to determine or even correct irradiation parameters.

For example, in one embodiment, there may be only two patient categories determined by a single recorded parameter, for example, patients younger than 50 years of age or older or light-skinned and dark-skinned patients. However, more than two, for example 3, more than 3, more than 5 or even more than 10 categories can be provided, whose respective assignment to a patient also depends on more than one parameter, for example at least two or at least three parameters.

Depending on the patient category assigned to the individual patient, in some cases already prefabricated planning data sets can be assigned, which for example provide for specific parameter sets for each individual or a group of area sections. In other cases, the planning data sets can still be created individually using saved defaults, especially patterns and rules, but the patterns and rules used can depend on the assigned patient category. In this way, the specifications can be made less complex.

It is also conceivable, depending on the patient category assigned to each patient, to limit certain categories of planning data, such as the laser parameters or target values for measurement data, which are measured during the laser treatment and which are influenced by the laser treatment. For example the target data may represent the current maximum permissible temperature of the retina at the treatment site during treatment, so that only a subset of possible parameters is available for the planning data sets. This simplifies and speeds up the creation of a planning dataset in many cases after assigning a specific patient category for an individual patient.

However, it is also conceivable to create certain patient categories for patients that are identified as problem categories and require special planning, for example, particularly careful or small-scale planning, or that preclude or make impossible the automatic or semi-automatic creation of a planning dataset. In this case, an assigned patient category could also refer to fully expert-based treatment planning and/or treatment.

For example, a patient category into which patients with highly pigmented skin and/or highly pigmented retina and/or a dark iris colour are assigned could be linked to the rule that certain laser power densities must not be exceeded.

For example, certain categories of patients with pre-existing conditions can be linked to the condition that certain temperatures of the retina, which are monitored by recording certain measurement data during the treatment, must not be exceeded. Alternatively, the target data corresponding to the desired temperature of the retina at the treatment site during treatment could also be selected depending on the patient category determined by previous diseases, known physiological data of the patient or pre-treatments. Similarly, the selection of location data for laser treatment, such as treatment area regions or area sections, and the selection of location data for areas excluded from laser treatment may also depend on the patient category.

A further embodiment of a device or system may provide that the inventory data acquisition device is arranged to acquire and provide diagnostic data and/or indication data and/or data of a performed pre-treatment and/or pre-known physiological data of the patient and/or already existing images of the retinal skin of the patient stored outside the system or device.

Such existing data can, for example, be in the form of an electronic file, can be entered manually or can be read in, for example a diagnosis in text or image form or an indication which already provides information on a recommended treatment. Such information may already contain data that differentiates certain locations or areas of the retina and is location-dependent on the respective location on the retina, such as the exact location of points or areas to be treated. However, such information can also be location-independent and describe the condition of the patient in general, his eyes, or other characteristics, such as physiological data of the patient. Such data may be in the form of older images of the retina of various types, for example colour images, black and white images, infrared images, fluorescence images or tomography images, or may consist of age information, skin colour or sex of the patient, an identity of the patient or information about pre-treatments which may have taken place with or without the involvement of the system according to the invention.

Further, in one embodiment, a device for laser treatment can be designed in such a way that a measurement data acquisition device is provided which has one or more sensor devices for acquiring and providing physiological data of the patient, in particular data which depend on the respective location on the retina and/or data which do not depend on a location on the retina, and that the processing device is set up to take into account the acquired physiological data of the patient when assigning planning data.

Such measurement data acquisition device, which can be integrated in a laser treatment device for tissue treatment of the retina according to the invention, can in principle be all kinds of devices which enable the current acquisition of additional information about the patient and his physiological data. By means of optical images of the retina, for example, algorithms can be used to detect retinal disease, which can be taken into account for treatment. A device for creating an OCT image or a device for reflectometry can also be provided. Sensor devices are also conceivable that measure other physiological characteristics of the patient, such as skin colour and/or brightness, skin thickness, hair colour, colour and/or colour distribution of the iris, blood pressure, various blood values or the like. These sensor devices may also include, for example, a camera or optical sensors.

Furthermore, in a system and a treatment device of the aforementioned type, it can be provided that the processing device has a storage device which is set up for storing inventory data of patients and planning data records assigned thereto, and in particular additionally for storing physiological data associated in each case with the aforementioned data and acquired by a measurement data acquisition device.

This enables the system or the treatment device to compare the assignment of a current planning data record to the inventory data of a patient with earlier assignments for similar or differing inventory data. This can be used, for example, to perform a plausibility analysis.

Furthermore, in such a system and a treatment device, it can be provided that the processing device has a storage device which is set up for storing inventory data of patients as well as structured image data generated with the system and/or images of the retina of the patient and planning data records assigned to these, in particular, inter alia, target values for measurement data, and, in particular, additionally for storing physical data respectively associated with the aforementioned data and recorded by a measurement data acquisition device and/or for storing manual changes made to the planning data records.

This embodiment makes it possible to compare the assignment of a current planning data record to the inventory data of a patient and the structured image data with earlier assignments for similar or differing inventory data and also stored structured image data. All these data can be assigned to or structured according to predefined area sections of the retina. A plausibility analysis can also be performed with these data. If manual planning changes have been made in earlier cases, these can also be taken into account and, for example, anticipated in the planning. In addition, if recorded physiological data are also stored in the expert system formed in this way, these can also be taken into account when comparing the planning file to be created with earlier planning.

In an implementation of the device or system, the planning data sets generated by the system can be assigned to or structured according to area sections and the planning data sets may contain planning data of one, two, three, four or five of the following categories: Laser parameters, location data for laser treatment, treatment patterns, location data for areas excluded from laser treatment, and target values for measurements taken during subsequent treatment by the measurement acquisition device or otherwise. Such target values can be, for example, an average target temperature or a target temperature depending on the location on the retina, which is continuously recorded during the laser treatment and which, for example, is to be reached, maintained or not exceeded. The other irradiation parameters can then be dynamically adjusted during the treatment so that the specified target values are reached or not exceeded. Thus, for example, parameters for a control system are specified in the planning data records.

It can further be provided that the processing device is designed as a self-learning device in which the assignment function, which assigns planning data to the inventory data and in particular to the structured image data and in particular to measured physiological data of a patient, is trained in such a way that it receives at least data from at least one of the following categories for a patient: Inventory data, structured image data, measured physiological data, and, on the other hand, associated planning data, in particular, inter alia, target values for measured data, as well as manual planning data changes and, in particular, data on an effect of the treatment recorded after implementation of the planned treatment are specified as training data.

The planning data sets generated by the system can contain planning data of one, two, three, four or five of the following categories: Laser parameters, location data, for example treatment area regions or area sections for laser treatment, treatment patterns, location data for areas excluded from laser treatment, target values for measurement data. In this context, the laser parameters may include, for example, laser power, wavelength, pulse duration, repetition rate of pulses, and total number of laser pulses, as well as the size of the light spot of the laser on the retina, and each of these or all conceivable sub-combinations. These laser parameters may depend on the location or area on the retina. They may also be uniform for one treatment area region or one of the predefined area sections at a time.

The areas of the retina excluded from treatment may include one or more areas of the retina. The treatment patterns can also be areas which, after a start impulse by the operator, are independently processed by the treatment laser spot by laser spot. The treatment patterns can also simply comprise treatment area regions or area sections which the laser works off in one go, for example point by point according to a point pattern, without the operator having to control each individual point. Only one starting point can be controlled e.g. automatically, and the rest of a treatment pattern can then be covered automatically by the laser controller. The density of the points to be approached can also be one of the parameters of the "treatment pattern" (patterns) category. Planning data can also be target values for measurement data acquired during treatment, such as a tissue temperature to be reached or not exceeded on the retina during treatment, which can be specified as an overall average value or location-dependent and which is continuously acquired/measured during treatment. The treatment system is then controlled using a real time retinal temperature measurement in such a way that the target values are reached or kept.

The areas to be excluded from treatment can play a special role. In many cases, they can be a complementary set of the location data for the laser treatment, but in many cases it is less error-prone to specify the areas of the retina to be excluded from the treatment than the location data for the treatment, especially, for example, when the retina is to be treated uniformly except for a few areas to be excluded.

The system can be designed as a self-learning system which, based on the above-mentioned data made available to it, continuously improves the assignment algorithm which generates planning data. In doing so, the system may have a neural network that is trained or an analytical, statistical or parametric learning algorithm, or may provide static, guided learning or self-learning. The assignment algorithm may additionally have analytical elements that are not changeable by the learning process and are predetermined with expert knowledge, such as certain conditions that the laser parameters must meet, for example power limits or prescriptions that the exclusions of treatment locations must follow.

In the following, the invention is shown by means of figures of a drawing and further described below.

Therein shows

Figure 1:     a device for tissue treatment of the retina with a laser according to the invention,

Figure 2:     a laser treatment device with a temperature control,

Figure 3:     a diagram about useful power density ranges for retina laser treatment,

Figure 4, 5, 6:     area sections of the retina that can be predefined,

Figure 7:     area sections with a treatment area region and laser spots located in the treatment area region, and

Figure 8:     a retina laser treatment device with an imaging device.

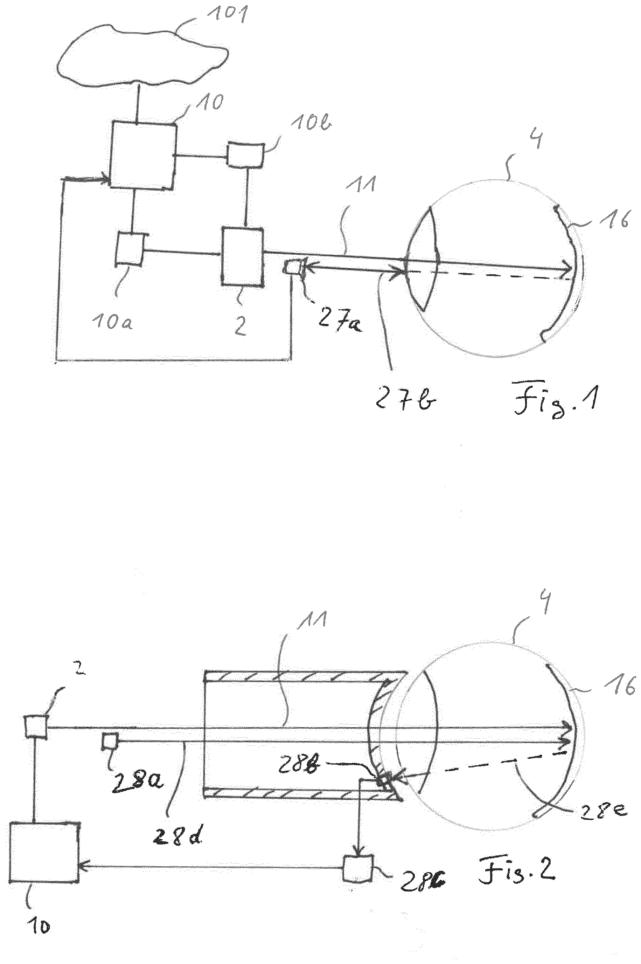

Figure 1 shows a device for tissue treatment of the retina 16 of a patient's eye 4 with a laser/treatment laser 2, which is emitting a laser beam 11 through the lens of the eye to the retina. Instead of a laser, in some cases also a different light source may be used like, e.g. a light emitting diode. A laser (or light source) control unit 10 generally controls the direction and intensity of the laser radiation and in some cases concretely parameters like the laser power, modulation frequency, duty cycle of the laser, the laser collimation, size of the laser spot and, in some cases, also the wavelength of the laser radiation. These parameters can be controlled by the laser control unit 10 in coordination with the more specific control unit 10a. Further, the deflection of the laser beam can be controlled via a deflection control unit 10b which in some cases controls one or more deflection mirrors or other comparable deflection means. The deflection control unit 10b enables a scanning of the surface area of the retina, for example by a laser spot pattern one laser spot after the other. An irradiation of one spot after the other may mean that the light beam jumps from spot to spot and remains on each spot for a certain time, but it could also, in some implementations of the invention, include a continuous movement from one spot to the next or a movement wherein faster and slower movement phases of the laser spot take turns.

The laser control unit is connected to a computer network/cloud 101 and/or to a remote server in order to exchange data, for example data of the patient who shall be treated. For example, data about the retina of the patient or other physiological data or personal data can be stored in the computer network along with a clearance for a general treatment with or without any special conditions. Further, parts of any computational efforts that could be necessary can be taken in the cloud as well as any kind of software updates.

The device further comprises an eye tracking device 27a, 27b with a camera 27a which is directed to the eye and is arranged to observe movements of the eye. For this purpose, in some cases a light beam 27b can be used which is sent to the eye and which is reflected on the surface of the eye, wherein the reflection is observed and analysed by the camera 27a and an analysing device. Information about the eye movement is then sent to the laser control unit 10 which can adapt the deflection of the laser beam 11 accordingly in order to keep the laser beam on a target spot.

Figure 2 shows a similar treatment device as Figure 1, wherein the laser control is simplified and only a Laser 2 is shown which is controlled by a laser control unit 10. A laser beam is directed to the retina 16 of an eye 4 for treatment, for example of a macula degeneration disease.

The control of the temperature which is reached on the retina by the treatment shall remain very reliably in a certain limited temperature window. This is in many cases reached by controlling the laser power as well as other parameters of the laser. The potential absorption of the laser light by the retina can be better assessed if the retina is further analysed by taking a picture with a camera or scanner. This method is described more in detail below.

Another method of keeping the temperature limits can be implemented by measuring the temperature of the retina at or very close to the current laser spot and controlling the laser power or other laser parameters based on this information. With the temperature measurement, a closed loop control can be established. This setup is shown in Figure 2 including the laser control unit 10.

The device for this purpose comprises a tissue temperature control mechanism with a tissue temperature measurement device 28a, 28b, 28c, which sends measurement values of the retina tissue to the laser control unit 10. This way, a closed loop control can be established which makes sure that the target temperatures can be reached and kept. The temperature measurement device is based on the "Grüneisen" formula and measures the absorption of short pulses of radiation sent to the retina by an excitation laser 28a by response signals in the form of pressure waves 28e which are generated by the sudden temperature rise on the retina which is created by pulses of a the radiation beam 28d of the excitation laser. The pressure waves are captured by a pressure wave transducer 28b. The signals generated by the pressure wave transducer 28b are transformed into temperature values by a transforming unit 28c.

The excitation laser 28a can be a laser which is separate from the treatment laser, but also, in some implementations, the treatment laser itself can be used as an excitation laser for the temperature measurement.

The temperature measurement device can be used to establish a closed loop with the laser control 10 and reliably limit the temperature at a current laser spot to the target temperature. For this purpose, the laser control unit 10 can control based on the measured temperature the laser power, the duty cycle, the laser spot diameter or even the wavelength of the treatment laser.

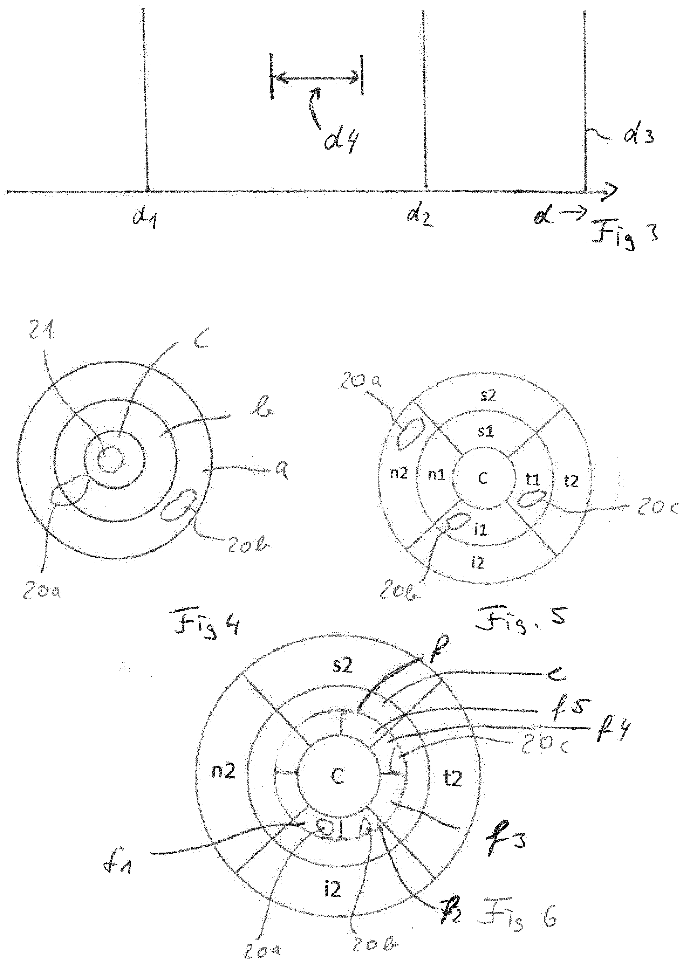

Figure 3 shows a diagram with different ranges of laser energy density d or radiation intensity on the horizontal axis.

The first vertical line at the value d1 is representing the official limit for eye security which is defined by IEC standard IEC 60825-1 at a value of 31 W/cm$^2$.

Laser power densities below this threshold are officially applicable without any risk.

The second vertical line represents d2, which is a power density well above 350W/cm$^2$. Above this value, laser treatment may cause light damages which can be proven with sensitive diagnostic methods, but in many cases are not immediately perceivable by the patient himself.

The third vertical line represents the value d3. Laser treatments with a power density above this value cause immediate damage to the retina, which is perceivable with standard methods.

The range d4 of power densities which is claimed according to the invention is located between d1 and d2. In this range, no damage is caused at the tissue by the laser radiation, but useful physiological processes in the retina tissue are initiated, enhanced, accelerated, or at least influenced in a positive way.

Figures 4 to 6 are showing different compositions of area sections on the retina of an eye. Figure 4 simply shows two concentric circular rings a and b which are surrounding a central circular area c. The rings a and b can be area sections of the retina, whereas the area c is not covered by the area sections and shall not be irradiated because the fovea 21 is located in this central area. Two treatment area regions 20a, 20b are shown, wherein the treatment area region 20b is located in the area section a and the treatment area region 20a partially extends to both area sections a and b.

Figure 5 shows the standard ETDRS grid with an outer circle, which has four area segments i2, n2, s2, t2 forming ring segments and an inner circle with the four area sections i1, n1, s1, t1 also forming ring segments. Figure 5 further shows three treatment area regions 20a, 20b, and 20c, wherein the treatment area region 20a is located in the area section n2, the treatment area region 20b is located in the area section i1 and the treatment area region 20c is located in the area section t1.

Figure 6 shows a different potential composition of area sections, wherein an outer circular ring similarly to the composition shown in Figure 5 is divided into segments forming area sections i2, n2, s2, t2, while the inner ring is subdivided into two smaller concentric rings e and f. The outer ring e is divided into 4 area segments similar to the segments of the segments i2, n2, s2, t2, while the inner ring f is equally divided into eight ring segments each of them forming an area segment.

By this finer subdivision, the area segments, which are closer to the fovea are smaller and thus, in this region close to the fovea, which is very sensitive to potential damages, the treatment area regions can be located by assignment to area sections more accurately or more in detail.

In the example of Figure 6, the treatment area regions 20a, 20b and 20c are assigned to the area segments f1, f2 and f4.

According to the invention, a rule for irradiation of the treatment area regions my state that the treatment area regions are treated starting at f1 proceeding counter clockwise. A potential rule may also state that all those area sections containing treatment area regions will be irradiated completely, also in a counter clockwise direction or in a clockwise direction. A rule can also state in the case that between two area sections which will be irradiated (in the example of Figure 6 the area sections f2 and f4) only one area section is located which has no treatment area region (in the example of Figure 6 the area section f3) this area section will not be left out and will be irradiated anyway. However, if between two area sections which carry treatment area regions, there are at least two area sections which do not carry treatment area regions, in other words, which are not planned to be irradiated, these area sections will be skipped.

By this rule, a quick proceeding is achieved and only in single cases additional area sections will be irradiated.

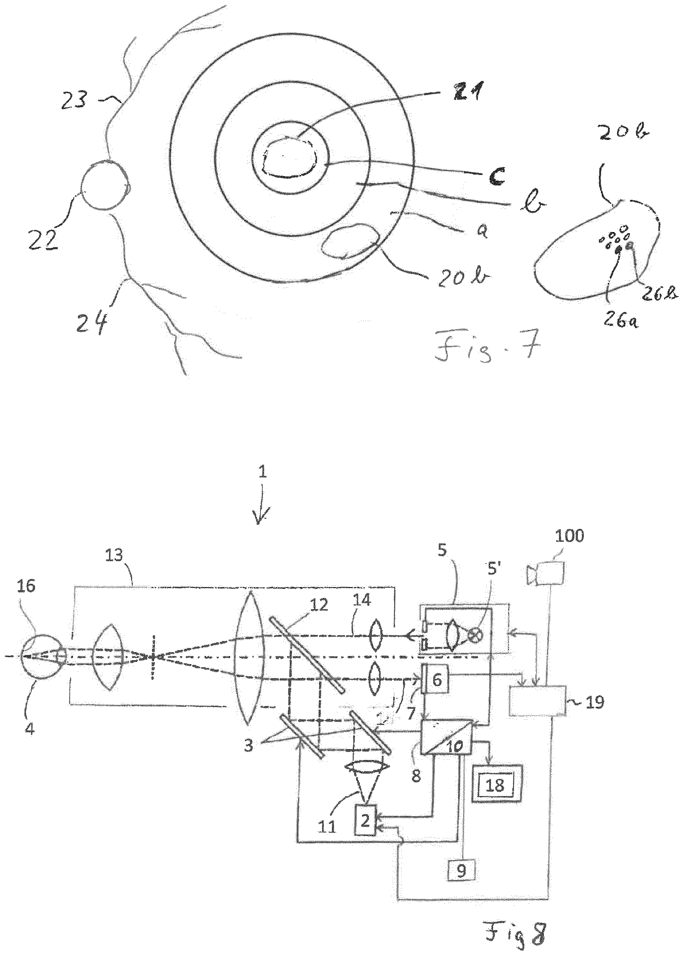

Figure 7 is showing the general setup of the area sections covering a part of the retina.

On the left side of the Figure, the optical nerve head 22 is shown at the position where it exits into the retina. From the same point, the major/primary arteries, called arcades 23, 24 are starting which support the retina with blood. The area of the retina which is covered by the area sections a, b in Figure 7 is defined by the fovea 21, which is located in the central circular area c, and by the arcades 23, 24 and the optical nerve head 22, which are located outside the outer ring a and thus, outside of the area sections.

On the right side of figure 7, it is shown as an example, how a treatment area region 20b is covered by several laser spots 26a, 26b which form a regular pattern. Patterns can be defined in advance, for example covering each of the area sections, because their shape is known and independent of any individual retina.

Figure 8 shows a device in the form of an ophthalmoscope or a device combining functions of an ophthalmoscope with functions of laser treatment of the retina 16 of a human eye 4.

The device in addition to the laser treatment part of the device, which is necessary for a general and potentially standardized treatment, has an illumination device 5 with a radiation source 5', which is arranged to direct an illumination beam 14 onto the eye 4 and the retina 16 of a patient. This allows the retina 16 to be suitably illuminated for capturing an image or a camera image under standardized and reproducible conditions. This allows in some cases of laser treatment to modify standard treatment parameters for a patient according to an individual measurement within certain boundaries.

The illumination may, for example, be equipped with a light emitting diode or an infrared diode as a light source, or with a light source of another type that provides a defined wavelength spectrum. The light source may also be, for example, a UV light source.

The ophthalmoscope also has a camera 6 in which a sensor 7 is provided. The sensor may be, for example, a CCD or CMOS sensor. Instead of the camera 6, any other type of device may be provided, for example a scanning device suitable for detecting radiation reflected or scattered from the retina.

The objective in operating the illumination device 5 and the camera 6 or an equivalent device is to obtain, under defined illumination conditions, the most accurate spatially resolved measurement data possible from the retina 16 by detecting a recording of backscattered radiation, and thus to be able to detect or determine the characteristics of target areas to be treated.

The illumination beam 14 and the reflected radiation 15 are suitably collimated and focused by a suitable optical system 13 with mirrors and lenses in a well-known manner. The optical system 13 also has a beam splitter 12, which makes it possible to direct a laser beam from the treatment laser 2 onto the retina 16 using the same optical axis as the illumination beam. Alternatively, the laser beam can be coupled without a beam splitter, for example by guiding it slightly laterally offset with respect to the illumination light. A control unit 8 can be provided for controlling the laser 2, wherein the control unit 8 on the one hand controls, for example triggers, the illumination device 5, and on the other hand acquires a camera image from the camera 6 and controls the laser 2 via a laser control unit 10. The laser control unit 10 can control the laser intensity of the laser 2 as well as deflection mirrors 3, which direct the beam path of the treatment beam 11 and thus enable the targeted treatment of individual treatment area regions (20a, 20b, 20c) on the retina 16.

For improved control of the laser 2, a processing device 19 is provided which permits precise processing of recordings/camera images of the camera 6 and links these to the known and defined parameters of the illumination of the retina 16.

The ophthalmoscope further comprises a sensor 100, for example in the form of a camera, which allows the measurement of the pigmentation colour and pigmentation intensity of the skin, the hair and/or the iris of the patient. An input device may also be provided to allow such measured parameters to be introduced into the system. In any case, these parameters are sent to the processing device 19, where they are taken into account in determining the values or correction values for irradiation parameters.

By illuminating the retina with known illumination parameters and linking them to the image of the retina, it is possible to assign, if necessary, correction factors for the intensity of the laser treatment for each treatment area region 20a, 20b, 20c on the retina 16 by the processing device 19 to the treatment area regions or area sections in an objectified manner. The intensity of the laser beam is therein given by the energy of the laser, the size of the laser spot on the retina and the number, repetition rate and duration of the pulse or pulses and the length of the pauses between the pulses (duty cycle of the laser).

In many areas of ophthalmology, different energy sources, particularly lasers, are used for diagnostics and treatment. As a rule, the entire beamed-in energy is absorbed by the biological tissue and converted to heat, the resulting temperature increase achieving the desired treatment effect. For example, during laser photocoagulation, the retina of the eye is thermally coagulated in a targeted manner. In the case of the conventional irradiations with irradiation times about 100 ms, temperatures of above 60° C occur. Also, in the case of transpupillary thermotherapy (TTT), temperature increases are utilized for achieving a vascular occlusion. In the case of photodynamic therapy (PDT), a previously injected dye is activated by laser irradiation of a therapy laser on the ocular fundus. The active ingredient develops its effect only on those cells to which it is bound. In this case also, almost the entire beamed-in energy is absorbed in the dye and in the retina and is converted to heat. During the respective irradiation time (pulse duration) with relatively long treatment and radiation pulses in the order of from $\mu$s to several hundred seconds, a temperature increase of the treated biological tissue, particularly of the fundus of the eye, which results in unintended damage to regions of the retina, should in any case be avoided. A non-invasive real-time temperature determination during ophthalmologic laser treatment has been desired for a long time. As a solution, a technology has been developed, which has already been shortly described with reference to Figure 2 and which allows to monitor the tissue temperature during the thermal treatment of biological tissue, in particular during ophthalmologic treatments, by means of optoacoustic techniques. In more detail, it is known to determine the tissue temperature of biological tissue caused by a laser treatment with optoacoustic techniques by pulsed laser irradiation. Temperatures are measured at the ocular fundus for example during the selective micro photo coagulation or during any other treatment of diseases of the retina which uses an irradiation source like, for example, a therapy laser. A special laser pulse which is intended for temperature measurement generates a thermal tissue expansion or, at the end of the pulse, a thermal tissue contraction, wherein the expansion as well as the contraction generate pressure waves and wherein the amplitude or other characteristics of the pressure waves can be used for calibrating the pressure wave characteristics, e.g., the amplitude, to the temperature or to a more complex control value with a defined relation to the temperature and to an absorption characteristic of the ocular fundus. From the change of subsequent pressure wave characteristics after subsequent treatment pulses, the temperature increase or decrease and, in addition, the respective absolute temperatures can be determined by means of the relation between the temperature of the tissue and the pressure wave characteristics, in particular the pressure wave amplitude, which is defined by the Grüneisen coefficient. It is an aspect of the current invention to provide a simplified method and a device for the non-invasive determination of the control value mentioned above or of the temperature on treated biological tissue, which can also be an implementation of a control value. The control value or the measured temperature may then, as already explained above, serve for controlling the power of the therapy laser. The control value may for example be the current temperature of the treated tissue of the eye, which can be determined based on the measurement of the strength of the opto- acoustic pulses or the detected pressure waves. For example, the strength of the pressure waves, their amplitude, average amplitude or accumulated energy may be measured or determined.

The measured strength of the pressure waves depends on the tissue temperature, in which the pressure waves are generated, the absorption of the dedicated laser signals on their way to the tissue and the intensity of absorption of the laser signals in the tissue (hence, for example: the pigmentation strength). In the range of parameters, where the treatment generally takes place, a variation of the strength of the detected pressure waves dP is determined by the following equation:

$$dP = \mu \, \Gamma \, dH,$$

wherein dP is the Variation of the strength of the pressure waves, $\mu$ is the absorption strength of laser light in the tissue, $\Gamma$ is the Grüneisen coefficient and dH is the accumulated laser beam energy in the tissue (heat dissipation in the tissue has been neglected for short time ranges). Because both parameters, the tissue temperature and the absorption strength of the tissue, point into the same direction (high temperature and high absorption strength require less power of the therapy laser), the measured strength of the pressure waves is an appropriate control value for controlling the power of the therapy laser. Therein, it is one option to use the control value as such for the control of the therapy laser, but it is another option to first determine the current temperature of the tissue, for example based on a calibration, and after that to control the therapy laser based on the determined temperature value.

**Claims**

1. Device for tissue treatment of the retina (16) of a patient's eye (4) by means of a light source, in particular a laser (2) controllable with respect to one or more irradiation parameters, wherein the light beam (11) can be directed to different areas of the retina, wherein the device is adapted to assign irradiation parameters to selected treatment area regions (20a, 20b, 20c) of the retina and to irradiate these treatment area regions using the irradiation parameters, wherein each treatment area region is associated with one of a plurality of mutually adjacent, predefined area sections (n1, s1, t1, i1, n2, s2, t2, i2).

2. Device according to claim 1, **characterized in that** the device is arranged for the light source (2) to successively irradiate the predefined area sections (n1, s1, t1, i1, n2, s2, t2, i2) in a predetermined order or according to a predetermined rule.

3. Device according to one of the claims 1 or 2, **characterized in that** the light source control is set up to irradiate a group of area sections (n1, s1, t1, i1, n2, s2, t2, i2), in particular all area sections individually one after the other, in each case either not covering an area section at all or covering 100% of the area section.

4. Device according to one of claims 1 to 3, **characterized in that** at least one group of area sections (n1, s1, t1, i1, n2, s2, t2, i2) are arranged next to one another in the circumferential direction around the fovea and/or **in that** the area sections together cover an area of the retina surrounding the fovea (21), the extent of which is in particular such that it does not cover the exit site of the optic nerve (22) and in particular the arcades (23, 24).

5. Device according to any one of claims 1 to 4, **characterized in that** the area sections (n1, s1, t1, i1, n2, s2, t2, i2) are distributed around the fovea, in particular in the form of segments of at least one circular ring concentrically surrounding the fovea or of an elliptical ring surrounding the fovea.

6. Device according to one of the claims 1 to 5, **characterized in that** the area sections (n1, s1, t1, i1, n2, s2, t2, i2) are arranged on one or more rings (a, b), in particular circular rings, each of them concentrically surrounding the fovea (21), the area sections being arranged rotationally symmetrically with an n-count rotational symmetry, where n is in particular between 1 and 9 and is further in particular 2, 4 or 6.

7. Device according to one of claims 1 to 6, **characterized in that** the area sections (n1, s1, t1, i1, n2, s2, t2, i2) form ring segments of two or three con-centric circular rings (a, b).

8. Device according to one of claims 1 to 7, **characterized in that** the irradiation intensity of the light source, in particular the laser (2) is controlled in such a way that the power density in the treatment area regions (20a, 20b, 20c) irradiated by the light source is between 100 W/cm$^2$ and 350 W/cm$^2$, in particular between 120 W/cm$^2$ and 300 W/cm$^2$ , wherein the irradiation time for each light spot (26a, 26b) in a treatment area region remains less than 500 msec, in particular less than 200 msec.

9. Device according to one of claims 1 to 8, **characterized by** an eye-tracking device (25) which compensates for movements of the eye (4) during the irradiation of the treatment area regions (20a, 20b, 20c) and/or by a temperature measurement device comprising a thermo- optic spectrometer (28a, 28b, 28c) which sends laser pulses to the retina and registers pressure waves being emitted by the retina as a response signal.

10. Device according to any one of claims 1 to 9, **characterized by** a retina detection device (6, 7) which records an image of the predefined area sections (n1, s1, t1, i1, n2, s2, t2, i2) of the retina by means of an imaging process and assigns irradiation parameters associated with the individual area sections (n1, s1, t1, i1, n2, s2, t2, i2) or treatment area regions (20a, 20b, 20c) arranged thereon.

11. Method for tissue treatment of the retina (16) of an eye (4) of a patient by means of a light source, in particular a laser (2) controllable with respect to one or more irradiation parameters, wherein the light beam (11) can be directed to different areas of the retina, wherein firstly treatment area regions (20a, 20b, 20c) are defined or determined in which light irradiation is useful and thereafter the treatment rules, in particular the treatment order of treatment area regions are determined according to the assignment of the treatment area regions to predefined area sections (n1, s1, t1, i1, n2, s2, t2, i2) of the retina, wherein the predefined area sections are mutually adjoining one another and together covering an area surrounding the fovea (21), the extent of which is dimensioned in such a way that it does not cover the exit location of the optic nerve (22) and in particular the arcades (23, 24).

12. Method according to claim 11, **characterized in that** an image of the predefined area sections (n1, s1, t1, i1, n2, s2, t2, i2) of the retina (16) is recorded by an imaging method and irradiation parameters are assigned to the individual area sections or to treatment area regions (20a, 20b, 20c) defined on these.

13. Method according to claim 11 or 12, **characterized in that** during the irradiation of the treatment area regions (20a, 20b, 20c) a movement of the eye (4) is compensated by an eye-tracking method.

Fig. 1

Fig. 2

Fig 3

Fig 4

Fig. 5

Fig 6

Fig. 7

Fig 8

Europäisches
Patentamt

European
Patent Office

Office européen
des brevets

# EUROPEAN SEARCH REPORT

**Application Number**

EP 23 19 8979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/116672 A1 (YEE KINGMAN [US]) 9 May 2013 (2013-05-09) * paragraphs [0004], [0026], [0027], [0032], [0033], [0049], [0052], [0053], [0058], [0062] * * figure 5c * | 1-13 | INV. A61F9/007 A61F9/008 |
| X | US 2006/100677 A1 (BLUMENKRANZ MARK S [US] ET AL) 11 May 2006 (2006-05-11) * paragraphs [0001], [0016], [0017], [0024], [0036] * * figures 6b,6g,6h * | 1-8 | |
| X | US 2014/228824 A1 (YEE KINGMAN [US] ET AL) 14 August 2014 (2014-08-14) * paragraphs [0025], [0026], [0033], [0049], [0052], [0057], [0058] * * figures 5A-5F, 6c * | 1-6,8-10 | |

**TECHNICAL FIELDS SEARCHED (IPC)**

A61F

The present search report has been drawn up for all claims

| Place of search | Date of completion of the search | Examiner |
|---|---|---|
| Munich | 15 March 2024 | Jansen, Birte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 19 8979**

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2024

| Patent document cited in search report | | | Publication date | Patent family member(s) | | | Publication date |
|---|---|---|---|---|---|---|---|
| US 2013116672 | A1 | | 09-05-2013 | CN | 103997948 | A | 20-08-2014 |
| | | | | EP | 2768378 | A1 | 27-08-2014 |
| | | | | JP | 5882486 | B2 | 09-03-2016 |
| | | | | JP | 2014534011 | A | 18-12-2014 |
| | | | | US | 2013110092 | A1 | 02-05-2013 |
| | | | | US | 2013110093 | A1 | 02-05-2013 |
| | | | | US | 2013110206 | A1 | 02-05-2013 |
| | | | | US | 2013116672 | A1 | 09-05-2013 |
| | | | | US | 2016128871 | A1 | 12-05-2016 |
| | | | | US | 2016228295 | A1 | 11-08-2016 |
| | | | | US | 2017304119 | A1 | 26-10-2017 |
| | | | | WO | 2013059564 | A1 | 25-04-2013 |
| US 2006100677 | A1 | | 11-05-2006 | AU | 2004311625 | A1 | 21-07-2005 |
| | | | | DK | 1701651 | T3 | 19-01-2015 |
| | | | | EP | 1701651 | A2 | 20-09-2006 |
| | | | | ES | 2526402 | T3 | 12-01-2015 |
| | | | | FI | 9630 | U1 | 19-04-2012 |
| | | | | IL | 176393 | A | 30-12-2010 |
| | | | | JP | 4377405 | B2 | 02-12-2009 |
| | | | | JP | 2006524515 | A | 02-11-2006 |
| | | | | US | 2006100677 | A1 | 11-05-2006 |
| | | | | US | 2010249760 | A1 | 30-09-2010 |
| | | | | US | 2010256615 | A1 | 07-10-2010 |
| | | | | US | 2014081248 | A1 | 20-03-2014 |
| | | | | US | 2016262933 | A1 | 15-09-2016 |
| | | | | WO | 2005065116 | A2 | 21-07-2005 |
| US 2014228824 | A1 | | 14-08-2014 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1875857 A1